# EUROPEAN PATENT APPLICATION

(11) **EP 1 632 485 A1**
(43) Date of publication of application: **08.03.2006**
(21) Application number: 04020959.5
(22) Date of filing: 03.09.2004
(51) Int. Cl.: C07D 307/91, C07D 209/88

(54) **Method of producing 3,4,5,7,8,9-hexahydro-2h-dibenzofuran-1-one**

(71) Applicant: SK Corporation, Seoul 110-110 (KR)
(72) Inventor: Kwak, Byong-Sung, Yuseong-gu Daejeon 305-761 (KR); Chung, Ki-Nam, Yuseong-gu Daejeon 305-761 (KR); Lee, Sang-il, Yuseong-gu Daejeon 305-728 (KR); Jeon, Seon-Yeong, Daejeon 306-826 (KR)
(74) Representative: Cattaneo, Elisabetta

(57) **Abstract**

Disclosed is a process of producing 3,4,5,7,8,9-hexahydro-2H-dibenzofuran-1-one, and 1-hydroxydibenzofuran and 4-hydroxycarbazole using 3,4,5,7,8,9-hexahydro-2H-dibenzofuran-1-one as an intermediate. 2-chlorocyclohexanone and 1,3-cyclohexanedione are reacted with each other in a base and organic solvent to form a carbon-carbon bond, and then subject to a cyclization reaction in the presence of an acid solution to produce 3,4,5,7,8,9-hexahydro-2H-dibenzofuran-1-one. 3,4,5,7,8,9-hexahydro-2H-dibenzofuran-1-one as the intermediate is subjected to nitrogen substitution and dehydrogenation reactions to produce 4-hydroxycarbazole. 1-hydroxydibenzofuran is produced by dehydrogenating 3,4,5,7,8,9-hexahydro-2H-dibenzofuran-1-one. The process according to the present invention is advantageous in that a low-priced starting material is used to improve economic efficiency, the reaction is stable in specific base and organic solvent conditions, and 3,4,5,7,8,9-hexahydro-2H-dibenzofuran-1-one is produced at high yield because of the cyclization reaction in the presence of the acid solution. Furthermore, 1-hydroxydibenzofuran and 4-hydroxycarbazole can be stably and economically produced using the above intermediate.

## Description

### BACKGROUND OF THE INVENTION

### 1. Field of the invention

The present invention relates to a method of producing 3,4,5,7,8,9-hexahydro-2H-dibenzofuran-1-one, and 1-hydroxydibenzofuran and 4-hydroxycarbazole using 3,4,5,7,8,9-hexahydro-2H-dibenzofuran-1-one as an intermediate. More particularly, the present invention pertains to a method of producing 1-hydroxydibenzofuran and 4-hydroxycarbazole, in which 2-chlorocyclohexanone and 1,3-cyclohexanedione react with each other in a base and organic solvent condition to form a carbon-carbon bond and are subjected to a rapid cyclization reaction in an acidic condition to produce 3,4,5,7,8,9-hexahydro-2H-dibenzofuran-1-one, oxygen of 3,4,5,7,8,9-hexahydro-2H-dibenzofuran-1-one is substituted by nitrogen to produce 1,2,3,5,6,7,8,9-octahydrocarbazole-4-one, and 3,4,5,7,8,9-hexahydro-2H-dibenzofuran-1-one and 1,2,3,5,6,7,8,9-octahydrocarbazole-4-one are dehydrogenated in the presence of a high boiling point solvent and a metal catalyst to produce 1-hydroxydibenzofuran and 4-hydroxycarbazole.

### 2. Description of the Prior Art

As an example of a process of producing 3,4,5,7,8,9-hexahydro-2H-dibenzofuran-1-one used as an intermediate of 1-hydroxydibenzofuran and 4-hydroxycarbazole according to the present invention, J. Chem. Soc. Perkin trans 1984, 6, p.1213 discloses a process of synthesizing 3,4,5,7,8,9-hexahydro-2H-dibenzofuran-1-one, in which 1,3-cyclohexanedione and cyclohexanone are recycled using paratoluene sulfonic acid under a xylene condition. However the process has a disadvantage in that the yield is merely 14%.

Meanwhile, Organic Letter, 2000. 2. 10., p.1387 discloses a process of synthesizing 3,4,5,7,8,9-hexahydro-2H-dibenzofuran-1-one, which includes reacting 1,3-cyclohexanedione with phenylsulfanyl cyclohexene using a Fetizon's reagent (Ag₂CO₃/Celite). However, Ag₂CO₃ is uneconomical for industrial use.

According to another process of producing 3,4,5,7,8,9-hexahydro-2H-dibenzofuran-1-one as described in J. Indian. Chem. Soc. 1962, 39, p.599, 1,3-cyclohexanedione reacts with 2-chlorocyclohexanone in potassium hydroxide and methanol. However, this process has a disadvantage of a relatively low yield of 53 %. With respect to this, the present inventors reacted 1,3-cyclohexanedione with 2-chlorocyclohexanone in the presence of sodium hydroxide, methanol, and water under optimum composition ratio conditions, resulting in a slightly increased yield of 55 %, but they found that since 2-chlorocyclohexanone is unstable in a basic condition, it is difficult to increase the yield under the above reaction conditions.

1-hydroxydibenzofuran and 4-hydroxycarbazole according to the present invention are intermediates of dibenzofuranyloxy alkanoic acids/esters and carbazolyloxy alkanoic acids/esters known as anti-cholesterol substances lowering a serum lipid concentration as described in US Pat. No. 3,846,445. Additionally, 4-hydroxycarbazole is also known as an intermediate of carvedilol, which is a nonselective beta blocker manufactured by Roche Ltd., and is used as an important intermediate of other medical supplies.

A method of synthesizing 4-hydroxycarbazole is classified into a method employing bromonitrobenzene and iodoanisole, and a method employing chloronitrobenzene and dinitrobiphenyl, as disclosed in Germany Pat. No. 2240599 by Boehringer Mannheim. However, in the above patent, since very uneconomical and dangerous hydrazoic acid (HN₃) is used as a starting material, there is a difficulty in its industrial use.

Meanwhile, in US Pat. No. 4,273,711, there is disclosed a process of producing 4-hydroxycarbazole. According to this patent, cyclohexanedione reacts with phenylhydrazine to give cyclohexane-1,3-dione-monophenylhydrazone which is converted by a Fischer rearrangement using zinc chloride into 1,2,3,4-tetrahydro-4-oxo-carbazole, dehydrogenation of which gives 4-hydroxycarbazole in a nickel catalyst. However, US Pat. No. 4,273,711 is disadvantageous in that acetic acid is used, causing a pungent odor, yield is poor, and a dehydration reaction time is excessively long, and thus, the process is uneconomical for the industrial production of 4-hydroxycarbazole.

### SUMMARY OF THE INVENTION

Therefore, the present invention has been made keeping in mind the above problems occurring in the prior art, and an object of the present invention is to provide a method of producing 3,4,5,7,8,9-hexahydro-2H-dibenzofuran-1-one used as an intermediate at a high yield, in which a specific base is used so as to stabilize 2-chlorocyclohexanone as a starting material and an acid solution treatment is carried out after formation of a carbon-carbon bond.

Another object of the present invention is to provide a method of effectively producing 1-hydroxydibenzofuran and 4-hydroxycarbazole in a more environmentally friendly manner than a conventional process, using an environmentally friendly raw material that is inexpensive and neither pungent nor hazardous, as a starting material.

The above objects can be accomplished by providing a method of producing 3,4,5,7,8,9-hexahydro-2H-dibenzofuran-1-one represented by Formula 3, which includes (a) reacting 2-chlorocyclohexanone, represented by Formula 1, with 1,3-cyclohexanedione, represented by Formula 2, in a base and organic solvent to form a carbon-carbon bond, and (b) cyclizing a resulting substance in the presence of an acid solution.

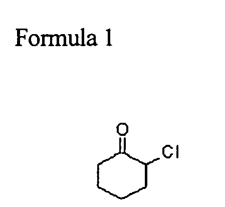

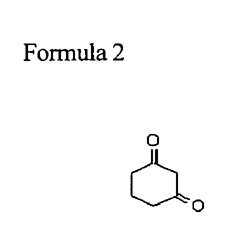

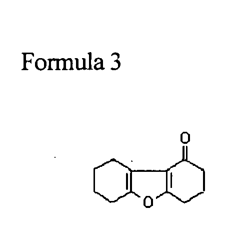

Furthermore, the present invention provides a method of producing 1-hydroxydibenzofuran represented by Formula 5, which includes (a) reacting 2-chlorocyclohexanone, represented by Formula 1, with 1,3-cyclohexanedione, represented by Formula 2, in a base and organic solvent to form a carbon-carbon bond, and acidifying a resulting substance using an acid solution to produce 3,4,5,7,8,9-hexahydro-2H-dibenzofuran-1-one, represented by Formula 3, through a rapid cyclization reaction, and (b) dehydrogenating 3,4,5,7,8,9-hexahydro-2H-dibenzofuran-1-one in the presence of a high boiling point solvent and a metal catalyst.

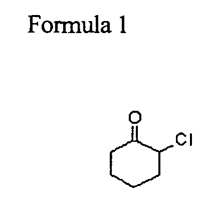

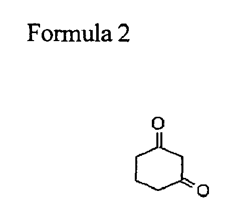

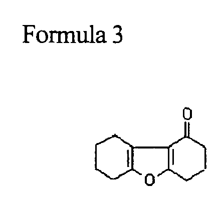

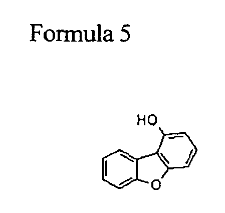

Additionally, the present invention provides a method of producing 4-hydroxycarbazole represented by Formula 6, which includes (a) reacting 2-chlorocyclohexanone, represented by Formula 1, with 1,3-cyclohexanedione, represented by Formula 2, in a base and organic solvent to form a carbon-carbon bond, and acidifying a resulting substance using an acid solution to produce 3,4,5,7,8,9-hexahydro-2H-dibenzofuran-1-one, represented by Formula 3, through a rapid cyclization reaction, (b) substituting nitrogen for oxygen of 3,4,5,7,8,9-hexahydro-2H-dibenzofuran-1-one using ammonia or ammonia solution to produce 1,2,3,5,6,7,8,9-octahydrocarbazole-4-on represented by Formula 4, and (c) dehydrogenating 1,2,3,5,6,7,8,9-octahydrocarbazole-4-on in the presence of a high boiling point solvent and a metal catalyst.

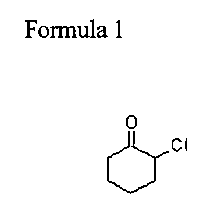

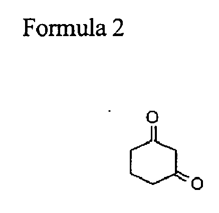

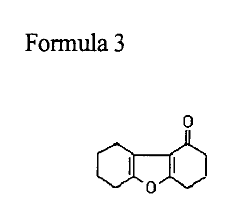

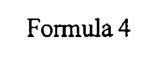

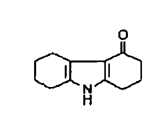

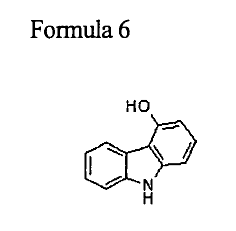

### DETAILED DESCRIPTION OF THE INVENTION

Hereinafter, a detailed description will be given of the present invention.

Examples of a base used in a reaction for producing 3,4,5,7,8,9-hexahydro-2H-dibenzofuran-1-one according to the present invention include sodium hydroxide, potassium hydroxide, potassium carbonate, sodium carbonate, sodium bicarbonate, calcium hydroxide, magnesium hydroxide, lithium hydroxide, pyridine, and trimethylamine, and particularly it is preferable to use potassium carbonate. The reason for this is that use of potassium carbonate increases the stability of the reaction.

A organic solvent may be selected from the group consisting of acetone, methyl ethyl ketone, methyl isobutyl ketone, normal hexane, normal heptane, benzene, toluene, xylene, diethyl ether, di-n-butyl ether, methyl-neo-butyl ether, isopropyl ether, tetrahydrofuran, dichloromethane, chloroform, dichloroethane, methanol, ethanol, butanol, isobutyl alcohol, isopropyl alcohol, acetonitrile, dimethylformamide, and a mixture thereof, and it is preferable to use acetone, methyl ethyl ketone or methyl isobutyl ketone.

Meanwhile, in the course of forming a carbon-carbon bond, a reaction temperature is within a range of 0 to 150°C, and preferably 40 to 80°C, and a reaction time is within a range of 1 to 6 hours. When the reaction temperature is lower than 0°C, the reaction time is lengthened and economic efficiency is poor, and when the reaction temperature is higher than 150°C, byproducts may be generated. When the reaction time is less than 1 hour, the reaction does not occur, and when the reaction time is more than 6 hours, economic efficiency is poor.

After the formation of the carbon-carbon bond is completed, acetone is removed, and reactants are dissolved in water and then acidified with an acid solution, such as hydrochloric acid, sulfuric acid, phosphoric acid, nitric acid, or acetic acid. Such an acidification brings about a rapid cyclization reaction, thereby producing 3,4,5,7,8,9-hexahydro-2H-dibenzofuran-1-one at a yield of about 85 %.

Hereinafter, a description will be given of a process of producing 4-hydroxycarbazole using 1,2,3,5,6,7,8,9-octahydrocarbazole-4-one after production of 1,2,3,5,6,7,8,9-octahydrocarbazole-4-one using 3,4,5,7,8,9-hexahydro-2H-dibenzofuran-1-one, and another process of producing 1-hydroxydibenzofuran using 3,4,5,7,8,9-hexahydro-2H-dibenzofuran-1-one. In this regard, a description of the production of 3,4,5,7,8,9-hexahydro-2H-dibenzofuran-1-one in the course of producing 4-hydroxycarbazole and 1-hydroxydibenzofuran will be omitted because it is the same as the above description.

To begin with, the production of 4-hydroxycarbazole will be described.

When 3,4,5,7,8,9-hexahydro-2H-dibenzofuran-1-one reacts with a predetermined, equivalent or greater, amount of ammonia or ammonia solution, oxygen of 3,4,5,7,8,9-hexahydro-2H-dibenzofuran-1-one is substituted by nitrogen, thereby producing 1,2,3,5,6,7,8,9-octahydrocarbazole-4-one. With respect to this, in the case of using ammonia, it is preferable to use a solvent capable of dissolving reactants and products, and the solvent is at least one selected from the group consisting of normal hexane, normal heptane, benzene, toluene, xylene, diethyl ether, di-n-butyl ether, methyl neobutyl ether, isopropyl ether, tetrahydrofuran, 1,4-dioxane, diglyme, methanol, ethanol, butanol, isobutyl alcohol, isopropyl alcohol, and a mixture thereof

An equivalent ratio of ammonia to 3,4,5,7,8,9-hexahydro-2H-dibenzofuran-1-one is preferably 2 to 30. When the equivalent ratio is lower than 2, conversion efficiency is reduced, and when the equivalent ratio is more than 30, economic efficiency is not assured.

Meanwhile, it is preferable to conduct the substitution at 50 ― 250°C for 2 ― 60 hours, and when the temperature and time deviate from the above range, economic efficiency is reduced. After the completion of the reaction, a temperature of the reactant is reduced to 25°C or less to solidify the reactant, thereby producing 1,2,3,5,6,7,8,9-octahydrocarbazole-4-one of Formula 4 without an additional refining step.

1,2,3,5,6,7,8,9-octahydrocarbazole-4-one is dehydrogenated in the presence of a high boiling point solvent and a metal catalyst to produce 4-hydroxycarbazole. A solvent used in the dehydrogenation is at least one selected from the group consisting of cumene, trimethyl benzene, trichloro benzene, methyl naphthalene, dimethyl naphthalene, diglyme, triglyme, and a mixture thereof, and it is preferable to use a solvent having a boiling point of 180°C or more, such as trichloro benzene, methyl naphthalene, dimethyl naphthalene, or triglyme. If a reaction temperature is low, conversion efficiency is reduced, resulting in poor reactivity, and thus, it is required to use a high boiling point solvent. Meanwhile, it is preferable that examples of the metal catalyst used in the dehydrogenation include palladium-carbon, palladium-alumina, palladium-silica, or palladium-calcium carbonate.

It is preferable to conduct the dehydrogenation at 140 ― 350°C for 2 ― 60 hours, and when a reaction temperature is lower than 140°C, reactivity is reduced, and when the reaction temperature is higher than 350°C, selectivity is reduced. Furthermore, when a reaction time deviates from the above range, selectivity is reduced and economic efficiency is reduced.

3,4,5,7,8,9-hexahydro-2H-dibenzofuran-1-one is dehydrogenated in the presence of a high boiling point solvent and a metal catalyst to produce 1-hydroxydibenzofuran. In the dehydrogenation, detailed descriptions of the high boiling point solvent, metal catalyst, reaction temperature and time are omitted in the specification because they are the same as those in the case of producing 4-hydroxycarbazole.

A better understanding of the present invention may be obtained through the following examples which are set forth to illustrate, but are not to be construed as the limit of the present invention.

### EXAMPLE 1: Production of 3,4,5,7,8,9-hexahydro-2H-dibenzofuran-1-one

After 1,3-cyclohexandion (100 g) and potassium carbonate (134 g) were put in a reaction vessel, 50 g of acetone was added and a mixture was agitated. 2-chlorocyclohexanone (118 g) slowly dropped in the reaction vessel. After the completion of dropping of 2-chlorocyclohexanone, the resulting mixture was heated to 0 ― 150°C, and agitated for 5 hours while being recycled. When the reaction was completed, acetone was removed, 200 g of water was added into the reaction vessel, agitation was carried out, and hydrochloric acid (6 N) was slowly added into the reaction vessel to acidify a solution in the reaction vessel. At this time, the acidification was continued until the pH was about 2, the acidified solution was extracted with dichloromethane, and the solvent was removed. The resulting solid was dissolved in a small amount of methanol and then dropped into water to produce 3,4,5,7,8,9-hexahydro-2H-dibenzomran-1-one (145 g) having a purity of 99 %.

### EXAMPLE 2: Production of 1,2,3,5,6,7,8,9-octahydrocarbazole-4-one

3,4,5,7,8,9-hexahydro-2H-dibenzofuran-1-one (100 g), produced according to example 1, and ammonia solution (600 g, assay 25 % as NH₃) were put in a high pressure batch reactor, and air in the reactor was replaced with nitrogen. When air was completely replaced with nitrogen, the reactor was heated to 130°C while strong agitation was conducted. After 20 hours, a progress of the reaction was checked, and the reactor was cooled to room temperature to precipitate 1,2,3,5,6,7,8,9-octahydrocarbazole-4-one in a solid phase. 1,2,3,5,6,7,8,9-octahydrocarbazole-4-one precipitated in the solid phase was filtered to produce 1,2,3,5,6,7,8,9-octahydrocarbazole-4-one having a purity of 98 % (84.1 g).

### EXAMPLE 3: Production of 4-hydroxycarbazole

1,2,3,5,6,7,8,9-octahydrocarbazole-4-one (50 g) produced according to example 2, a high boiling point aromatic solvent (b.p. = 230 ― 270°C, 300 g), and 5 % palladium-carbon catalyst (5 g) were put in a batch reactor. Hydrogen generated during a reaction was removed, and the reactor was heated to 230°C while nitrogen was fed into the reactor so as to prevent external air from flowing into the reactor. The reaction was continued until conversion efficiency of 1,2,3,5,6,7,8,9-octahydrocarbazole-4-on was 99 % or more while agitation continued and nitrogen was continuously fed into the reactor. After the completion of the reaction, the reaction solution was filtered at high temperatures, and the catalyst was removed. After the sodium hydroxide aqueous solution was added to remove the high boiling point aromatic solvent, the reactant was extracted using an aqueous solution layer and a product was neutralized with a hydrochloric acid solution to precipitate a solid. The solid was filtered to produce 4-hydroxycarbazole having a purity of 98 % (34 g).

### EXAMPLE 4: Production of 1-hydroxydibenzofuran

3,4,5,7,8,9-hexahydro-2H-dibenzofuran-1-one (5 g), produced according to example 1, a high boiling point aromatic solvent (b.p. = 230 ― 270°C, 300 g), and 5 % palladium-carbon catalyst (0.5 g) were put in a batch reactor. Hydrogen generated during a reaction was removed, and the reactor was heated to 230°C while nitrogen was fed into the reactor so as to prevent external air from flowing into the reactor. The reaction was continued until conversion efficiency of 3,4,5,7,8,9-hexahydro-2H-dibenzofuran-1-one was 99 % or more while agitation continued and nitrogen was continuously fed into the reactor. After the completion of the reaction, the reaction solution was filtered at high temperatures, and the catalyst was removed. After the sodium hydroxide aqueous solution was added to remove the high boiling point aromatic solvent, the reactant was extracted using an aqueous solution layer and a product was neutralized with a hydrochloric acid solution to precipitate a solid. The solid was filtered to produce 1-hydroxydibenzofuran having a purity of 98 % (3.87 g).

As described above, the present invention provides a reaction under specific base and acid conditions, and thus, 3,4,5,7,8,9-hexahydro-2H-dibenzofuran-1-one can be produced at high yield. Furthermore, since a substance which has a lower price than that used in conventional processes and is neither pungent nor hazardous is used as a starting material, 1-hydroxydibenzofuran and 4-hydroxycarbazole can be economically produced in an environmentally friendly manner.

## Claims

1. A method of producing 3,4,5,7,8,9-hexahydro-2H-dibenzofuran-1-one represented by Formula 3, comprising:
(a) reacting 2-chlorocyclohexanone, represented by Formula 1, with 1,3-cyclohexanedione, represented by Formula 2, in a base and organic solvent to form a carbon-carbon bond; and
(b) cyclizing a resulting substance in the presence of an acid solution.

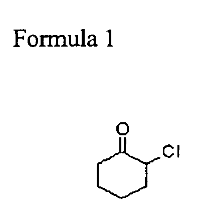

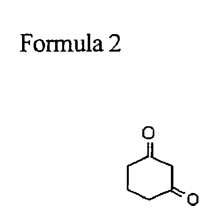

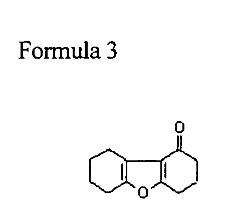

2. The method as set forth in claim 1, wherein the base is potassium carbonate.

3. The method as set forth in claim 1, wherein the organic solvent is selected from a group consisting of acetone, methyl ethyl ketone, methyl isobutyl ketone, and a mixture thereof.

4. The method as set forth in claim 1, wherein a reaction temperature is 0 ― 150°C and a reaction time is 1 ― 6 hours in the step (a).

5. The method as set forth in claim 1, wherein the acid solution is hydrochloric acid, sulfuric acid, phosphoric acid, nitric acid, or acetic acid.

6. A method of producing 1-hydroxydibenzofuran represented by Formula 5, comprising:
(a) reacting 2-chlorocyclohexanone, represented by Formula 1, with 1,3-cyclohexanedione, represented by Formula 2, in a base and organic solvent to form a carbon-carbon bond, and acidifying a resulting substance using an acid solution to produce 3,4,5,7,8,9-hexahydro-2H-dibenzofuran-1-one, represented by Formula 3, through a rapid cyclization reaction; and
(b) dehydrogenating 3,4,5,7,8,9-hexahydro-2H-dibenzofuran-1-one in the presence of a high boiling point solvent and a metal catalyst.

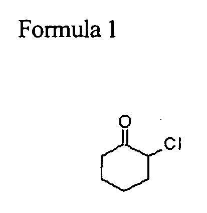

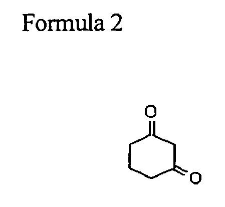

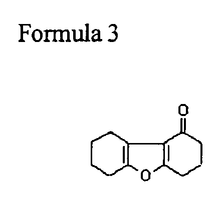

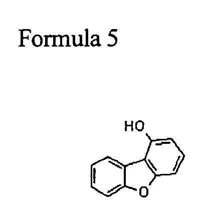

7. The method as set forth in claim 6, wherein the base is potassium carbonate.

8. The method as set forth in claim 6, wherein the organic solvent is selected from a group consisting of acetone, methyl ethyl ketone, methyl isobutyl ketone, and a mixture thereof.

9. The method as set forth in claim 6, wherein a reaction temperature and a reaction time required to form the carbon-carbon bond are 0 ―150°C and 1 ― 6 hours, respectively.

10. The method as set forth in claim 6, wherein the acid solution is hydrochloric acid, sulfuric acid, phosphoric acid, nitric acid, or acetic acid.

11. The method as set forth in claim 6, wherein the high boiling point solvent is selected from a group consisting of cumene, trimethyl benzene, trichloro benzene, methyl naphthalene, dimethyl naphthalene, diglyme, triglyme, and a mixture thereof

12. The method as set forth in claim 6, wherein the metal catalyst is palladium-carbon, palladium-alumina, palladium-silica, or palladium-calcium carbonate.

13. The method as set forth in claim, 6, wherein the dehydrogenating is conducted at 140 ― 350°C for 2 ― 60 hours in the step (b).

14. A method of producing 4-hydroxycarbazole represented by Formula 6, comprising:
(a) reacting 2-chlorocyclohexanone, represented by Formula 1, with 1,3-cyclohexanedione, represented by Formula 2, in a base and organic solvent to form a carbon-carbon bond, and acidifying a resulting substance using an acid solution to produce 3,4,5,7,8,9-hexahydro-2H-dibenzofuran-1-one, represented by Formula 3, through a rapid cyclization reaction;
(b) substituting nitrogen for oxygen of 3,4,5,7,8,9-hexahydro-2H-dibenzofuran-1-one using ammonia or ammonia solution to produce 1,2,3,5,6,7,8,9-octahydrocarbazole-4-one represented by Formula 4; and
(c) dehydrogenating 1,2,3,5,6,7,8,9-octahydrocarbazole-4-one in the presence of a high boiling point solvent and a metal catalyst.

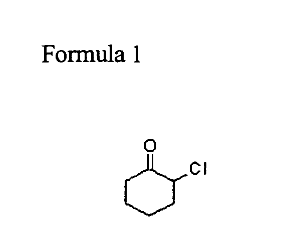

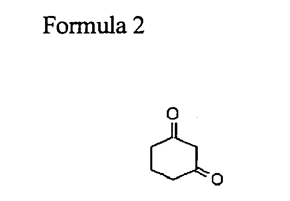

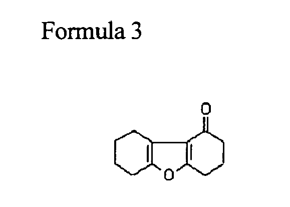

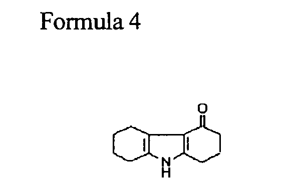

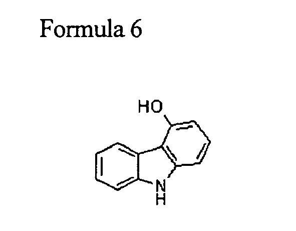

15. The method as set forth in claim 14, wherein the base is potassium carbonate.

16. The method as set forth in claim 14, wherein the organic solvent is selected from a group consisting of acetone, methyl ethyl ketone, methyl isobutyl ketone, and a mixture thereof

17. The method as set forth in claim 14, wherein a reaction temperature and a reaction time required to form the carbon-carbon bond are 0 ― 150°C and 1 ― 6 hours, respectively.

18. The method as set forth in claim 14, wherein the acid solution is hydrochloric acid, sulfuric acid, phosphoric acid, nitric acid, or acetic acid.

19. The method as set forth in claim 14, wherein an equivalent ratio of ammonia to 3,4,5,7,8,9-hexahydro-2H-dibenzofuran-1-one is 2 ― 30 in the step (b).

20. The method as set forth in claim 14, wherein a reaction temperature is 50 ― 250°C and a reaction time is 2 ― 60 hours in the step (b).

21. The method as set forth in claim 14, wherein the high boiling point solvent is selected from a group consisting of cumene, trimethyl benzene, trichloro benzene, methyl naphthalene, dimethyl naphthalene, diglyme, triglyme, and a mixture thereof.

22. The method as set forth in claim 14, wherein the metal catalyst is palladium-carbon, palladium-alumina, palladium-silica, or palladium-calcium carbonate.

23. The method as set forth in claim 14, wherein the dehydrogenating is conducted at 140 ― 350 °C for 2 ― 60 hours in the step (c).
